Europäisches Patentamt

European Patent Office

Office européen des brevets

(11) Publication number: **0 050 424**
**B1**

(12) **EUROPEAN PATENT SPECIFICATION**

(45) Date of publication of patent specification: **18.09.85**

(21) Application number: **81304411.2**

(22) Date of filing: **24.09.81**

(51) Int. Cl.⁴: **G 01 N 33/561,**
G 01 N 33/569,
G 01 N 33/571,
G 01 N 33/558

(54) **Method for producing an analytical antibody probe, an analytical antibody probe, and a method for analysing a sample for certain antibodies.**

(30) Priority: **24.09.80 US 190361**
**02.09.81 US 297962**

(43) Date of publication of application:
**28.04.82 Bulletin 82/17**

(45) Publication of the grant of the patent:
**18.09.85 Bulletin 85/38**

(84) Designated Contracting States:
**DE GB**

(56) References cited:
**EP-A-0 017 460**
**FR-A-2 233 024**
**GB-A-2 008 747**
**US-A-3 941 876**
**US-A-3 979 509**
**US-A-4 094 759**
**US-A-4 097 149**

(73) Proprietor: **CETUS CORPORATION**
**1400 Fifty-Third Street**
**Emeryville California 94608 (US)**

(72) Inventor: **Erlich, Henry Anthony**
**3936 Rhoda Avenue**
**Oakland California (US)**
Inventor: **Lovett, Michael Alan**
**20656 Pacific Coast Highway Apt. 2**
**Malibu California (US)**

(74) Representative: **Bizley, Richard Edward et al**
**BOULT, WADE & TENNANT 27 Furnival Street**
**London EC4A 1PQ (GB)**

(56) References cited:
**PROCEEDINGS OF THE NATIONAL ACADEMY OF SCIENCE, vol. 76, no. 9, September 1979 USA H. TOWBIN et al.: "Electrophoretic transfer of proteins from polyacrylamide gels to nitrocellulose sheets: Procedure and some applications" pages 4350-4354**

Courier Press, Leamington Spa, England.

**0 050 424**

(56) References cited:
CHEMICAL ABSTRACTS, vol. 92, no. 7, 18
February 1980, page 320, column 1, abstract
no. 54615x COLUMBUS, OHIO (US)

CHEMICAL ABSTRACTS, vol. 92, no. 1, 7
January 1980, page 276, column 1, abstract no.
2752v COLUMBUS, OHIO (US)

CHEMICAL ABSTRACTS, vol. 73, no. 11
September 14, 1970, page 66, column 2,
abstract no. 52950m COLUMBUS, OHIO (US)

Description

The present invention provides, in general, a method for producing an analytical antibody probe, an analytical antibody probe, and a method for analysing a sample for certain antibodies. The invention is used in the analysis of diseases or disease stages in patients.

Many clinical diagnostic techniques have, as their fundamental basis, the antigen-antibody reaction. This reaction serves as a defence against micro-organisms and other foreign bodies as part of the body's normal immunological response. Detection of the presence of antigen-antibody reactions in tests performed upon serum obtained from a patient may indicate the presence or absence of antibodies in the patient's serum. A positive test for reaction of antibody with a specific antigen may indicate a presence of a corresponding disease or at least suggest that diagnostic conclusion.

Known clinical diagnostic procedures that test for the antigen-antibody reaction in sera (serotests) may take a wide variety of forms. Some utilize antigens anchored to the surface of inert particles. In the presence of specific antibody, these particulate antigens clump visibly in an agglutination reaction. Such a procedure is widely employed in the diagnosis of syphilis and is known as the Venereal Disease Research Laboratory (VDRL) test. Other tests may involve the attachment of a fluorescent or radioactive moiety in such a way that its presence indicates that the antigen-antibody reaction has occurred. In a similar manner, enzymes have been linked as a detectable moiety — the so-called enzyme linked immunosorbent assay (ELISA).

The hallmark of conventional serotests is that they measure a single positive or negative signal. Signals may be, for example, hemagglutination, hemagglutination inhibition, complement fixation, surface fluorescence, particle agglutination and so on. That single positive or negative signal is usually a complex and undifferentiated array of the structural components of an organism or material antigenically related to it. This complexity of the antigen test material increases the likelihood that false positive signals will be generated due to antibodies stimulated by other organisms sharing some but not all antigenic properties with the test material. In contrast the present invention enables the provision of a diagnostic method and means by which the antigen-antibody reaction may be greatly expanded as a diagnostic tool. Using the invention the presence of a specific disease or a specific stage of a disease in a patient may be detected by a simple procedure.

U.S. Patent No. 3941876 is concerned with the determination, in vitro, of allergic hypersensitivity to a large number of allergens by the use of an apparatus comprising an elongated body of cellulosic material having separately applied thereto said allergens in respective narrow bands in a spaced apart relationship along the body.

When this apparatus is treated with appropriate antibody-containing blood serum antibody/antigen reactions occur and can be detected by using second and labelled antibodies directed against the first antibodies. This disclosure does not, however, unlike the present invention, involve predifferentiation of antigenic components material characteristic of a disease thereby to enable the resulting apparatus to provide a "fingerprint" test for that disease.

The present invention provides a method for producing an analytical antibody probe comprising disposing upon a solid support medium in a predetermined spatial relationship a set of differentiated antigenic components of a larger set of proteins and/or polysaccharides, said larger set being related to a disease or disease stage under analysis and said set of differentiated antigenic components being selected such that at least a subset thereof is known to be reactive with antibodies present in serum obtained from a patient having a specific disease or disease stage. The invention also provides an analytical antibody probe comprising a solid support medium upon which is disposed antigenic material related to a disease or disease stage, said antigenic material having been differentiated into distinct bands of antigenic components on said solid support medium in a relationship characteristic of said components.

One further aspect of the invention is the use of a probe produced by a method of the invention for analysing a sample to determine whether it contains antibodies specific for antigenic material characteristic of a particular disease or disease stage by contacting said probe with said sample under conditions which permit reaction of any said antibodies present in the sample with the differentiated antigenic components on the probe and detecting the existence and pattern of any antigen-antibody reactions on the probe, the probe antigenic components being related to the particular disease or disease stage under analysis.

The invention will now be further described with reference to the accompanying drawings, in which:—

FIGURE 1 is a photograph of a conventional SDS polyacrylamide gel showing the total protein profile of T. pallidum after staining;

FIGURE 2 is a photograph of an autoradiogram of twelve diagnostic strips of T. pallidum, constructed in accordance with the invention, after exposure to sera drawn from patients, eight of whom had different stages of syphilis and four of whom had exhibited false positive (BFP) on standard serological tests for syphilis;

FIGURE 3 is a photograph of an autoradiogram of nine diagnostic strips of T. pallidum, constructed in accordance with the invention, after exposure to sera drawn from patients suffering from late syphilis and secondary syphilis;

FIGURE 4 is a photograph of an autoradiogram of ten diagnostic strips of T. pallidum, constructed in accordance with the invention, after exposure to sera drawn from patients suffering from early latent and late latent syphilis;

FIGURE 5 is a photograph of conventional SDS gels showing the total protein profiles of five different immunotypes of *Chlamydia*;

FIGURE 6 is a photograph of an autoradiogram of five diagnostic strips of *Chlamydia trachomatis*, constructed in accordance with the invention, after exposure to rabbit antiserum to *Chlaymydia trachomatis* immuno type B;

FIGURE 7 is a photograph of an autoradiogram of seven diagnostic strips of *Toxoplasma gondii*, constructed in accordance with the invention, after exposure to sera drawn from patients with chronic and acute toxoplasmosis;

FIGURE 8 is a photograph of an autoradiogram of diagnostic strips of cytomegalovirus infected cells and cell free preparation of partially purified virus, constructed in accordance with the invention, after exposure to patient sera;

FIGURE 9 is a photograph of an autoradiogram of diagnostic strips of rabbit kidney cells infected with *Herpes simplex* virus I or II, constructed in accordance with the invention, after exposure to sera drawn from patients suffering from *Herpes* infections;

FIGURE 10 is a photograph of an autoradiogram of diagnostic strips of capsular polysaccharide types 4 and 8 for *Streptococcus pneumoniae*, constructed in accordance with the invention, after exposure to an immune serum;

FIGURE 11 is a photograph of an autoradiogram of a diagnostic strip of bee venom, constructed in accordance with the invention, after exposure to serum drawn from a patient allergic to bee venom;

FIGURES 12 and 13 are photographs of an autoradiogram of diagnostic strips of type $L_2$ *C. trachomatis*, constructed in accordance with the invention, after reaction with sera from patients with type F infection and type D infection, respectively;

FIGURE 14 is a photograph of an autoradiogram of diagnostic strips of *T. pallidum*, constructed in accordance with the invention, after exposure to sera drawn from patients suffering from syphilis and after exposure to IgG and IgM specific antisera probes;

FIGURE 15 is a photograph of an autoradiogram of diagnostic strips of *Toxoplasma gondii*, constructed in accordance with the invention, after exposure to serum drawn from a patient suffering from acute toxoplasmosis and after exposure to IgC and IgM specific probes;

FIGURE 16 is a photograph of an autoradiogram of diagnostic strips of cytomegalovirus, constructed in accordance with the invention, after exposure to a serum pool drawn from patients suffering from viral infection and after exposure to IgG and IgM specific probes;

FIGURE 17 is a photograph of an autoradiogram of diagnostic strips of *Toxoplasma gondii*, constructed in accordance with the invention, when the *Toxoplasma* antigens were separated on a nondenaturing isoelectricfocusing gel;

FIGURE 18 is a photograph of an autoradiogram of diagnostic strips of *Toxoplasma gondii*, prepared in accordance with the invention, when the *Toxoplasma* antigens are separated on a nondenaturing native gel system; and

FIGURE 19 is a photograph of an autoradiogram of a diagnostic strip of cloned *T. pallidum* — specific antigens, constructed in accordance with the invention, after exposure to sera from patients suffering from syphilis.

Very generally, the use of the invention for diagnosis involves a method comprising providing a solid-state substrate upon which is disposed in a predetermined relationship a set of differentiated antigenic components of a larger set of proteins or polysaccharides. The larger set is related to the disease or disease stage under diagnosis. The set of components is selected such that at least a subset of the components is known to be reactive with antibodies present in serum obtained from a patient suffering from the specific disease or disease stage. The substrate is contacted with serum obtained from the patient under diagnosis under conditions which permit reaction of antibodies in the serum with antigenic components on the substrate. The existence and pattern of antigen-antibody reactions on the substrate is then detected for correspondence with the pattern of those components on the substrate known to be reactive for the specific disease or disease stage.

The method detailed here for disease diagnosis differs markedly from conventional serotests in that what comprises a "positive" test is not the presence of *one* signal, but rather the appearance of a series of signals that represent antibody response to defined antigens associated with a specific pathogen or disease. The number and nature of the signals which define a disease state are defined empirically for each specific disease; all humans with this disease display the same multiple signals characteristic of that disease.

More particularly, the invention, although based upon the antigen-antibody reaction phenomenon, take advantage of a discovery of major significance. If protein or polysaccharide material related to a specific disease or stage of a disease is differentiated in such a way as to establish a spatial relationship of differentiated antigenic components, a plurality of antigen-antibody reactions may be detected upon exposure to serum from a diseased patient. The extent and pattern of such reactions, with proper differentiation, can be made highly specific to a given disease. Accordingly, a sort of molecular antigenic "fingerprint" may be established which will identify a particular disease and no other. Moreover, this molecular antigenic "fingerprint" may be designed so as to distinguish between historical antibodies, i.e., residual IgG antibodies which indicate previous exposure to a given antigen, and current antibody, i.e., recent IgM antibodies which indicate very recent exposure to a given antigen. In the case of allergy diagnosis, the antibody detected is immunoglobulin IgE.

The method of the invention utilizes a solid-state substrate upon which is disposed in a predetermined spatial relationship a set of differentiated antigenic components of a larger set of proteins or polysaccharides. This larger group is related to the disease or the stage of the disease under diagnosis. It may be the pathogen itself (e.g., *Herpes* virus I and II0 or it may be a tissue sample (e.g., of a sarcoma tumor) or it may be an allergen such as various types of grasses or pollen. Frequently, the source of the larger set of proteins or polysaccharides will itself be antigenic for a particular disease but, because of non-specificity, cross reactivity, or other problems, is unsatisfactory for diagnostic use.

The set of differentiated antigenic components of the larger set of proteins or polysaccharides is chosen for its specific ability to identify the disease or the stage of the disease of interest. These components are selected such that at least a subset of the components is known to be reactive with antibodies present in serum obtained from a patient having the specific disease or disease stage. The subsets are determined empirically in accordance with techniques described below. The process by which the components are differentiated depends upon the particular disease, the larger protein or polysaccharide from which the components are derived, and the particular antibodies which are to be detected (e.g., IgG, IgM, or IgE). Such differentiation processes may include, but are not limited to, electrophoresis (SDS or native gel), isoelectricfocusing, thin layer chromatography, and centrifugation. Another differentiation process, once the nature of the subject of components is ascertained, is to produce each component of the subset separately by means of genetically modified microorganisms. Each component may then be placed separately on the substrate.

In any case, the individual differentiated components are positioned upon a solid-state substrate such as a cellulose strip. The precise manner of attaching the components to the substrate will depend upon the nature of the components and the substrate. For example, if electrophoresis is utilized as a differentiation process, a useful transfer technique is the so-called filter affinity transfer as described by Erlich, H.A., et al., in *Journ. Biol. Chem.,* 254:12240—12247 (1979).

A typical solid-state substrate may be a cellulose strip to which a plurality of differentiated components of the pathogen responsible for a disease have been transferred. When the strip is exposed to a patient's serum, components which are reactive with antibodies in the serum bind to it and may be detected by any suitable assay. Before such exposure, the strip may be blank in appearance with the differentiated components not visible. Once exposed to antibodies, the antibody-antigen reactions cause the antibodies to bind to the strip in a pattern that is indicative of the present or absence of a specific disease. Through previous empirical testing, it can be readily established as to which differentiated proteins or groups of proteins on a given substrate will react with antibodies in the serum of a patient having the specific disease that is of interest. Thus, separate strips may be produced that are specific to, for example, *Chlamydia,* syphilis, and gonorrhea, respectively.

Once the strip or solid-state substrate is produced with the empirically determined and selected pattern of differentiated antigenic components established on the substrate, it is in a form useful for clinical diagnosis. For such a use, the substrate is exposed or contacted with serum obtained from the patient under diagnosis. The conditions under which the contact occurs are established so as to permit reaction of antibodies in the serum with antigenic components on the substrate.

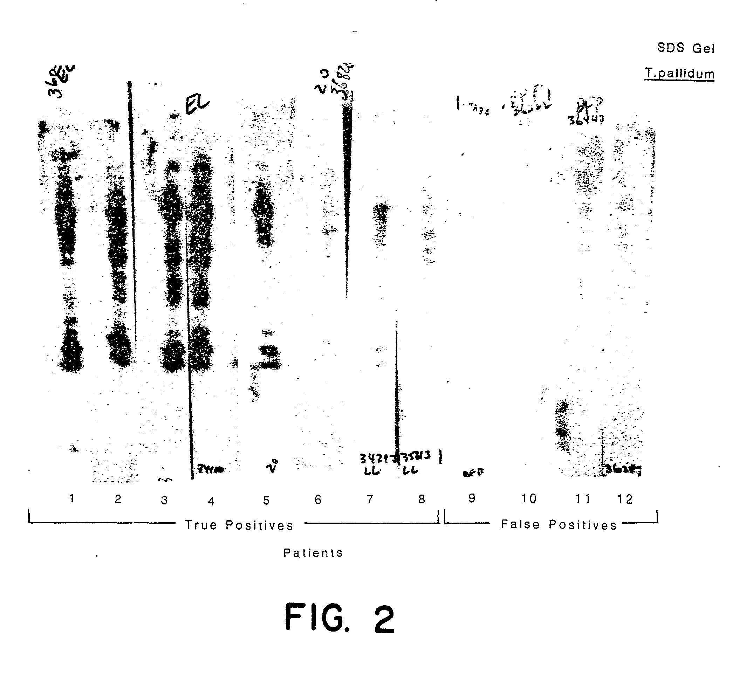

Following suitable illumination steps, e.g., radioactively labelled probes specific for human immunoglobulin classes and autoradiography, the strip is examined to ascertain the pattern, if any, of antibody-antigen reactions which has developed. If the pattern corresponds to the pattern known for the particular disease to which the strip or substrate corresponds, a positive diagnosis is obtained. Otherwise, the diagnosis is negative. Actual detection of the antigen-antibody reactions may employ other than autoradiographic assay of the type shown in FIGURE 2. For example, colorimetric assays may also be employed.

Traditionally, radioactively labelled *S. aureus* protein A has been used as a probe for IgG antibodies. To distinguish between reactions with historical antibody (IgG) and new antibody (IgM), antiserum to human IgM may be labelled in a variety of ways, e.g., $^{125}I$ or fluorescein, and used as probe to detect formation of new human antibody (IgM). This provides the ability to distinguish between persons with a history of a disease who do not currently have an active form of that disease and persons with the active form of the disease. Historical antibodies will, in many cases, remain with a cured person for life.

The present invention will be more readily understood by means of the following examples. These examples are set forth for the purpose of elaborating on the invention and are not intended to limit the invention in any way.

Example I. Use of the Invention for the Correct Diagnosis of Syphilis

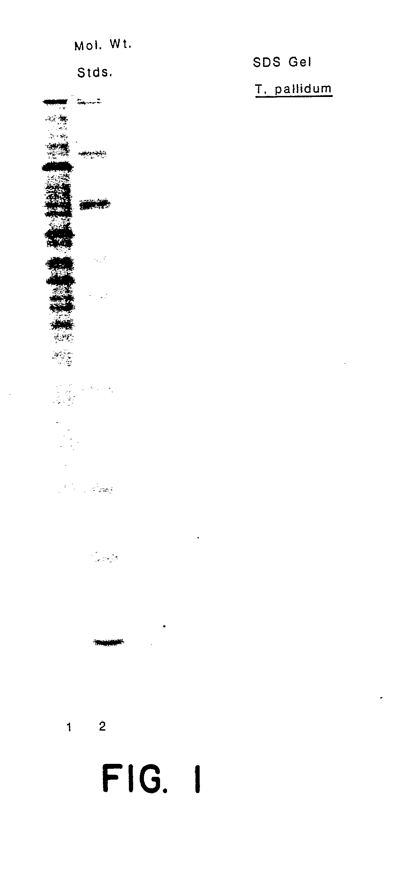

FIGURE 1 is a photograph showing the total protein profile of *Treponema pallidum.* These proteins are separated and stained on a conventional SDS polyacrylamide gel. To obtain this profile, intact *T. pallidum* was suspended in an electrophoresis sample buffer comprised of 62.5 mM tris (pH 6.8), 2% sodium dodecylsulfate, and 5% mercaptoethanol. The sample was then applied to an SDS polyacrylamide gel system as described in Laemmli, U.K., *Nature* (London) 227:680—685 (1970). The gel was run until the tracking dye reached the bottom of the gel.

In FIGURE 1, the left-hand column represents the *T. pallidum* profile, whereas the right-hand column is a system of molecular weight markers as is well known in the use of polyacrylamide gel separations. FIGURE 1 provides a base for comparison of the actual protein separation with the antigenic activity described, in FIGURE 2.

In FIGURE 2, diagnostic strips were prepared in accordance with the invention using the total protein separation of *T. pallidum* illustrated in FIGURE 1. To prepare the strips, the gel is overlaid with nitrocellulose paper as described by Towbin, H., Staehlin, T., and Gordon, J., *PNAS* (USA) 76:4350—4354 (1979). The paper is then covered with scouring pads and supported by lucite grids with numerous pores. The assembly is held together with rubber bands and is then placed in a single chamber for electrophoresis such that the surface of the gel applied directly to the paper is facing the anode. Electrophoresis is performed in an electrode buffer comprised of 25 mM tris, 192 mM glycine, and 20% volume/volume methanol at pH 8.3. Electrophoresis is carried out for 90 minutes. The nitrocellulose at the end of this procedure contains the proteins arrayed as they have been separated according to molecular weight and is referred to as the blot.

The blots were then soaked in a solution of 1% bovine serum albumin in a buffer comprised of 50 mM tris (pH 7.5), 0.9% sodium chloride, 0.25% gelatin, 0.2% sodium azide, and 0.1% NP 40 (TSGAN) for ten minutes at room temperature. This was to saturate all remaining reactive sites on the paper. At this point, the blots are ready for use and may be stored by freezing or other suitable means.

Each of the strips in FIGURE 2 is a blot of the *T. pallidum* total protein profile after exposure to patient serum representing different stages of syphilis (8 patients) and representing non-syphilitic patients who showed false positive (BFP) in standard serological tests for syphilis (4 patients). Serum dilutions were used at 1:1000 with twelve hours at room temperature with gentle shaking. After this period of incubation with serum, the blots were rinsed several times with TSGAN and then washed with TSGAN for 20—60 minutes at room temperature, again with gentle agitation. Then 2—4 microcuries of protein A with a specific activity of greater than $10^7$ counts per minute/microgram is added in a volume of 100—200 milliliters of TSGAN and incubation continued with a gentle agitation for 60 minutes at room temperature. A similar incubation buffer system is described in Renard, J., Reiser, J., and Stark, G. R., *PNAS* (USA) 76: 3116—312 (1979).

The blots were then rinsed several times with TSGAN, washed with TSGAN with gentle agitation at room temperature for 20 minutes and then rinsed several times with distilled water, dried with a hair dryer and then subjected to autoradiography. The autoradiography was on Kodak X-omat R film and DuPont Cronex intensifying screens. Autoradiography usually takes from 2—16 hours.

Of the strips for 8 syphilitic patients shown in FIGURE 2, it may be seen that similar reaction patterns exist, particularly with respect to patients 1—5 and 7. This is true also of patients 6 and 8, although the strength of the reactions is less pronounced. On the other hand, patients who were false positive in the VDRL test, shown in FIGURE 2 as patients 9—12, are clearly distinguishable from the true positives by the tests conducted in accordance with the invention.

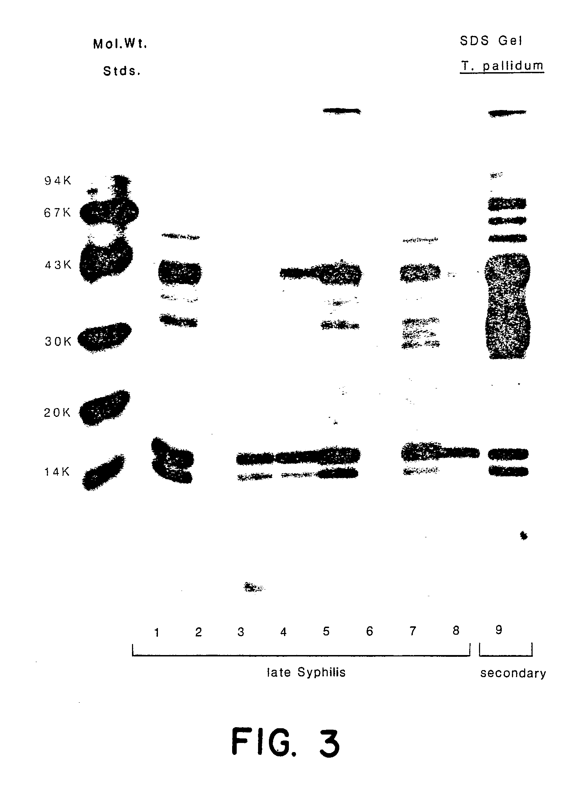

FIGURE 3 shows diagnostic strips of the IgG antibody response to peptides of *T. pallidum* from patients with late syphilis and secondary syphilis. The gels, protein samples and strips were prepared as outlined above. In FIGURE 3, the far-right strip is a system of molecular weight markers. Strips 1—8 illustrate the antibody response from patients suffering from late syphilis, while strip 9 shows the antibody response indicative of the secondary form of the disease.

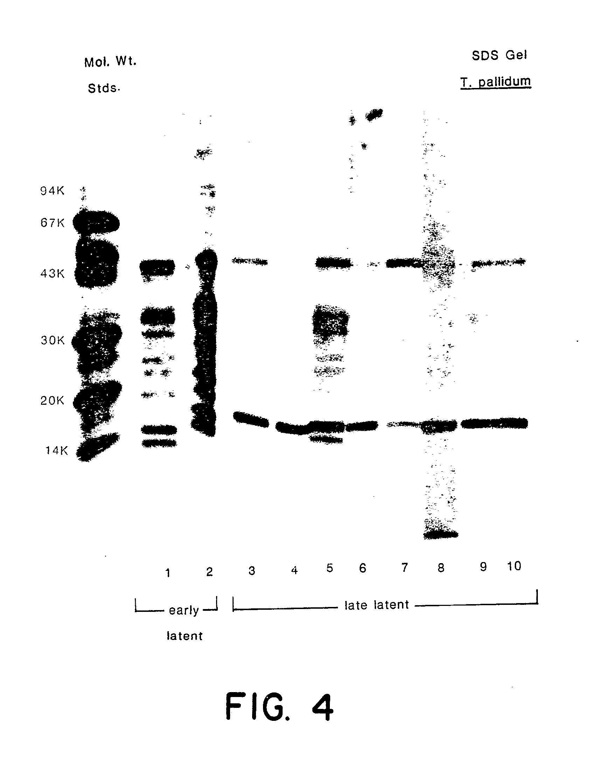

FIGURE 4 shows diagnostic strips of the IgG antibody response to peptides of *T. pallidum* from patients with early latent and late latent syphilis. Again the samples were prepared as outlined above, and again the far-right column is a system of standard molecular weight gel markers. Strips 1 and 2 demonstrate the response from patients in the early latent stage of the disease. Strips 3—10, which exhibit a different fingerprint pattern, are from patients in the late latent stage of the disease. It can thus be seen that the diagnostic strip of the invention provides a much more reliable test for indicating the presence of the various stages of syphilis than do the standard serological tests for syphilis.

### Example II. Use of the Invention to Detect *Chlamydia* Antigen

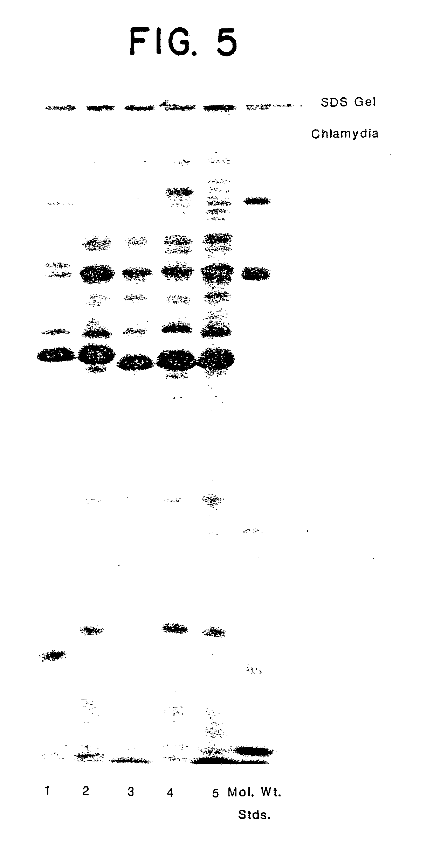

FIGURE 5, left-hand columns 1—5, show the stained total proteins of five different immunotypes of *Chlamydia*. Column 6 in FIGURE 5 is a molecular weight marker system. By standard serology, these immunotypes are non-cross-reactive. Accordingly, a separate antiserum for clinical use must be prepared for each serotype.

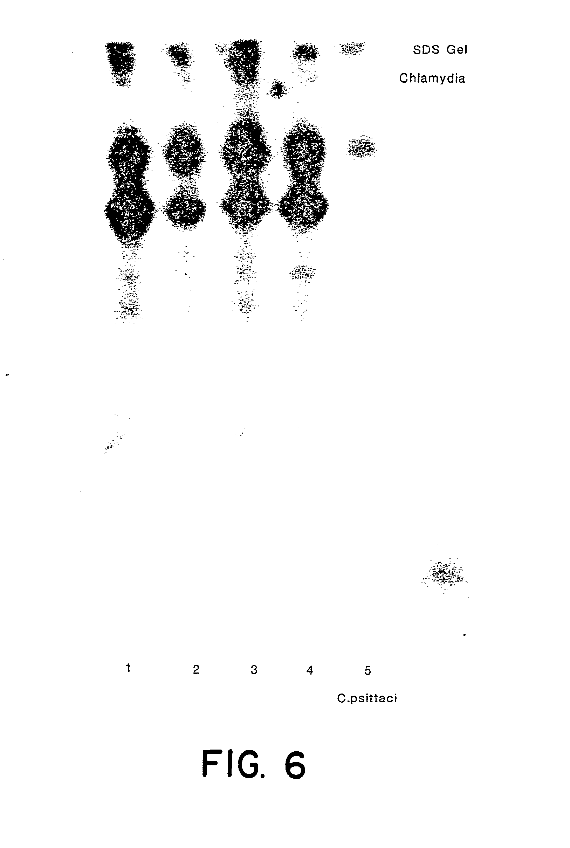

FIGURE 6 shows the strips of the invention prepared in accordance with Example I after exposure to rabbit antiserum to *Chlamydia trachomatis* immuno type B and autoradiography. Preparations and procedures were as in Example I. All *C. trachomatis* immuno types have extensive cross-reaction of the major antigenic proteins. It may be seen that the left-hand 4 strips show strong reaction whereas the strip in the far right-hand side, specific for *C. psittaci*, shows weak relatedness of only two antigens. This illustrates that a single *C. trachomatis* immunotype is a sufficient source of antigens for testing human infection with any other *C. trachomatis* immunotypes, and yet provides specificity in that other types of *Chlamydia* may be readily distinguished.

### Example III. Use of the Invention to Detect *Toxoplasma gondii* Antigen

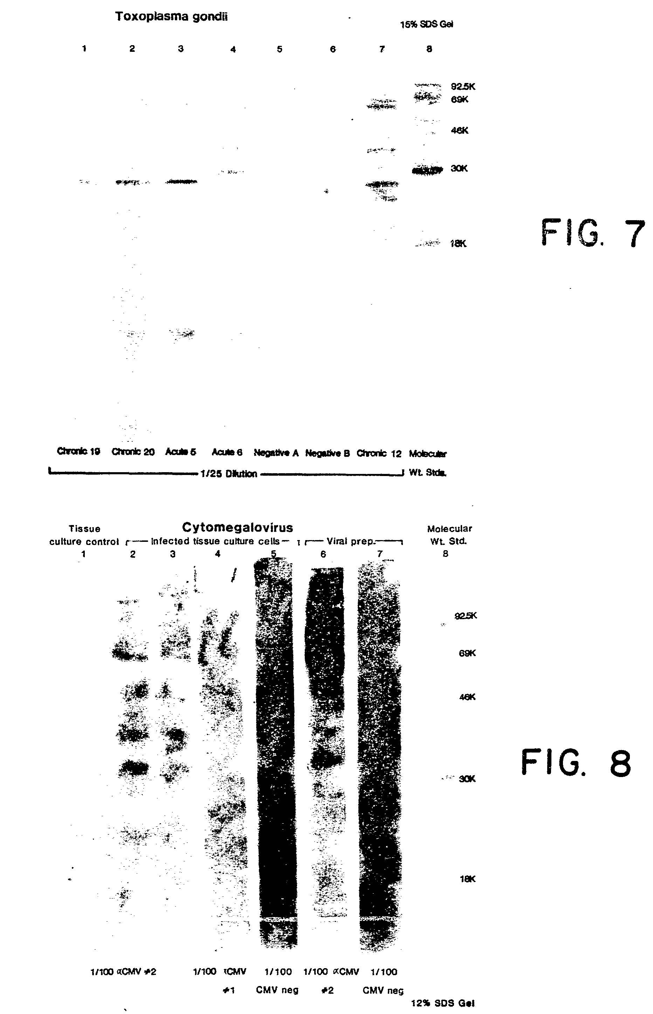

FIGURE 7 is a photograph showing various antigenic bands of *Toxoplasma gondii* that react with antibodies in the sera of patients with chronic and acute toxoplasmosis. To obtain the observed patterns, the toxoplasmal antigens were separated on a conventional SDS polyacrylamide gel. A sonicate of *Toxoplasma* was suspended in an equal volume of electrophoresis sample buffer consisting of 0.125M trizma base (pH 6.8), 2.5% sodium dodecylsulfate, and 2.5% β-mercaptoethanol. The sample was then applied to an SDS polyacrylamide gel system as described in Laemmli, U.K., *Nature* (London) 227:680—685 (1970). The gel was run until the tracking dye reached the bottom of the gel.

The gel was then washed for 15 minutes in water and in two 5-minute washes of 50 mM sodium acetate, pH 7.0. The peptide components of *Toxoplasma,* separated by molecular weight in the SDS polyacrylamide gel, were electrophoretically transferred onto cyanogen bromide activated paper as follows. The gel was placed on a scotch bright pad covered with filter paper. A sheet of cyanogen bromide treated filter paper was laid on the gel and another sheet of filter paper and a scotch bright pad was placed on top. The assembly is placed in an E—C electroblot unit with the cyanogen bromide paper facing the anode. Electrophoresis was carried out in 50 mM sodium actate, pH 7, at 25 volts for one hour.

All remaining reactive sites on the cyanogen bromide paper are bound and/or inactivated by soaking the paper in a solution of 1M glycine and 1% bovine serum albumin for 0.5 to 3 hours. The paper was washed three times for 5—15 minutes each with agitation in a wash solution containing 0.1% ovalbumin, 0.1% between 20, 0.02% sodium azide in phosphate buffered saline. The paper is then incubated at room temperature with gentle agitation for 2—3 hours in diluted human serum. The sera used in FIGURE 7 are from patients with chronic or acute toxoplasmosis. The patient's serum is diluted 1:25 in wash solution.

After the incubation with serum, the paper is washed three times for 5—15 minutes with shaking in wash solution. The $^{125}I$ Protein A is added to the paper using a 1:200 dilution of stock (~5 µg/ml, 15 µci/µg) in wash solution. The protein A is iodinated using the chloramine T method as described by Erlich, H., Cohen, S., and McDevitt, H., in *Cell*, 13:681—689 (1978). The paper is incubated with the $^{125}I$ Protein A for 1—3 hours at room temperature with agitation. The paper is again washed as above, dried and placed under Kodak XAR-5 X-ray film for 16 hours.

Strips 1, 2 and 7 in FIGURE 7 show the reaction of sera from patients suffering from chronic toxoplasmosis; strips 3 and 4 illustrate the acute form of the disease. Controls from uninfected patients are shown in strips 6 and 7. Collectively the strips show that use of the invention allows not only the detection of toxoplasmosis but also the ability to distinguish between chronic and acute forms of the disease.

Example IV. Use of the Invention to Detect Cytomegalovirus Antigen

The strips in FIGURE 8 illustrate the banding patterns obtained when cytomegalovirus infected cells and cell free preparation of partially purified virus are reacted with patient sera. The electrophoresis and transfer are performed as outlined in Example III. The preparation of the infected cells was done as follows.

Two Corning 490 cm² roller bottles containing a confluent layer of passage eight human embryo lung cells were each inoculated with 2.5 ml of infected cells containing between $10^7$ and $10^8$ viral particles/ml. Fourteen ml of Eagle minimal essential medium plus 10% fetal calf serum was added to each bottle. The cells were incubated at 37°C for 1.5 hours before addition of a further 93 ml medium. Seven days later the cells were trypsinised off and centrifuged down at 2,000 RPM for 5 minutes at room temperature. The resulting 1.5 ml of packed cells were resuspended in 3.5 ml of medium, frozen in dry ice and stored at −20°C for 13 days. The free virus is contained in the supernatant from infected cells. A control flask of uninfected human embryo lung cells was also prepared.

Example V. Use of the Invention to Detect *Herpes Simplex* Virus Antigen

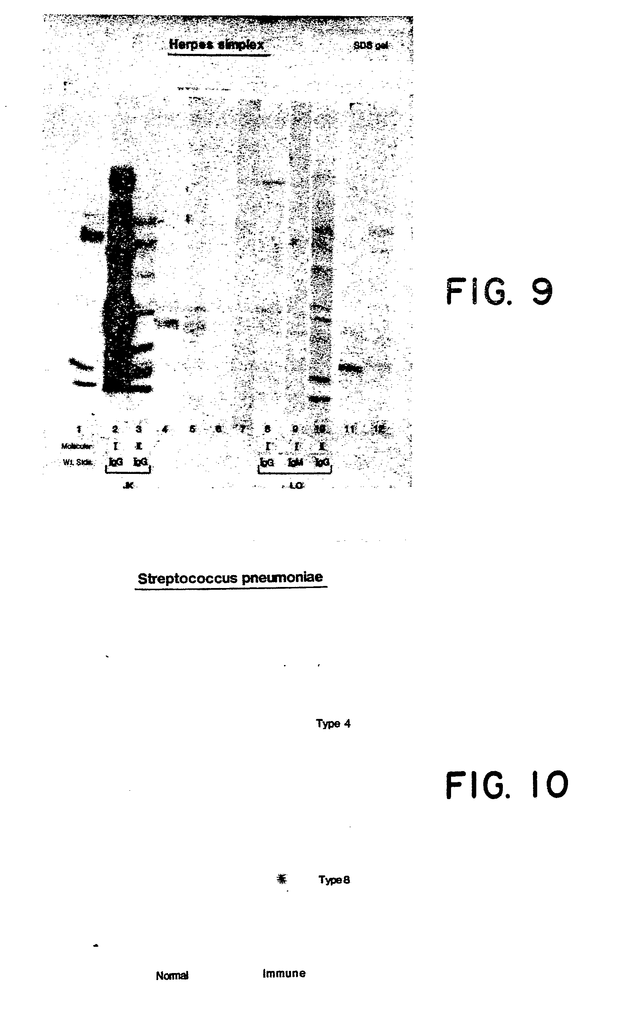

FIGURE 9 illustrates this Example. Rabbit kidney cells were infected with either *Herpes simplex* virus type I or *Herpex simplex* virus type II. Peptides from these infected cells were separated on 9.5% denaturing SDS polyacrylamide gels as described in Example I. Diagnostic strips derived from the gels were then cross-reacted with serum from patients suffering wtih type I or type II *Herpes* infections. FIGURE 9 is a photograph of an autoradiogram of these diagnostic strips following exposure to appropriate radioactive probes.

Strip 1, i.e., the far right-hand strip on the photograph, shows a standard system of molecular weight markers. Strips 2 and 3 show how serum from patient J.K. reacts with antigens from the two types of *Herpes*. Strip 2 represents proteins derived from *Herpes* virus type I while strip 3 represents proteins derived from *Herpes* virus type II. Comparison of the strips shows that serum from patient J.K. contains IgG antibodies that react strongly with the peptides derived from *Herpes* virus type I infected cells, and only weakly with peptides derived from *Herpes* virus type II infected cells.

Strips 8, 9 and 10 show the reaction of serum from patient L.O. Strips 8 and 9 represent proteins derived from *Herpes* virus type I; strip 8 proteins demonstrate an IgG reaction while strip 9 demonstrates the presence of IgM's. Strip 10 represents proteins derived from *Herpes* virus type II. Comparison of the strips shows that serum from patient L.O. contains IgG antibodies to proteins derived from *Herpes* virus type II infected cells.

They also show L.O.'s serum reacts only weakly with protein derived from *Herpes* virus type I infected cells. Strips 4—7 and strips 11 and 12 are not relevant to this Example.

Example VI. Use of the Invention for Detection of Polysaccharide Antigen

FIGURE 10 illustrates the reaction of immune serum with pneumococcal capsular polysaccharide of types 4 and 8. Three micrograms of purified polysaccharide were spotted onto two nitrocellulose strips. Pre-immune serum did not react with these antigens. Immune serum to type 8 reacted strongly with type 8 and less strongly with type 4 pneumococcal polysaccharides.

Example VII. Use of the Invention for Diagnosis of an Allergy

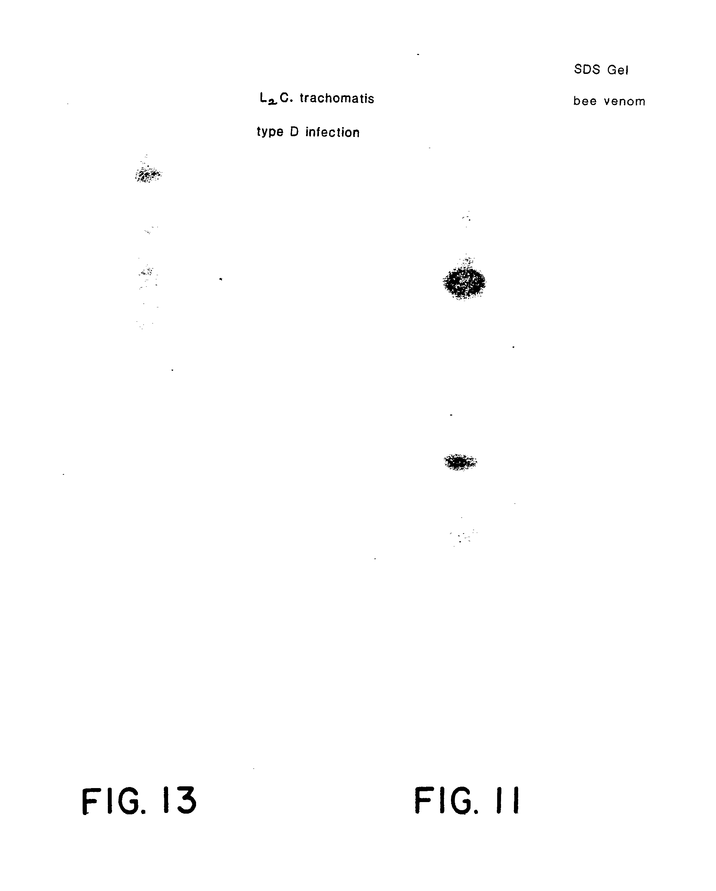

FIGURE 11 illustrates the reaction of sera from a patient allergic to bee venom. The strip was prepared using intact bee venom profiled on a SDS polyacrylamide gel in accordance with the previously described procedures. Serum dilutions were 1:20 with exposure for 18 hours at room temperature with gentle shaking. The probe used for autoradiography was $^{125}I$ rabbit antihuman IgE.

Example VIII. The Invention is Not Limited by Immunotype Specificity

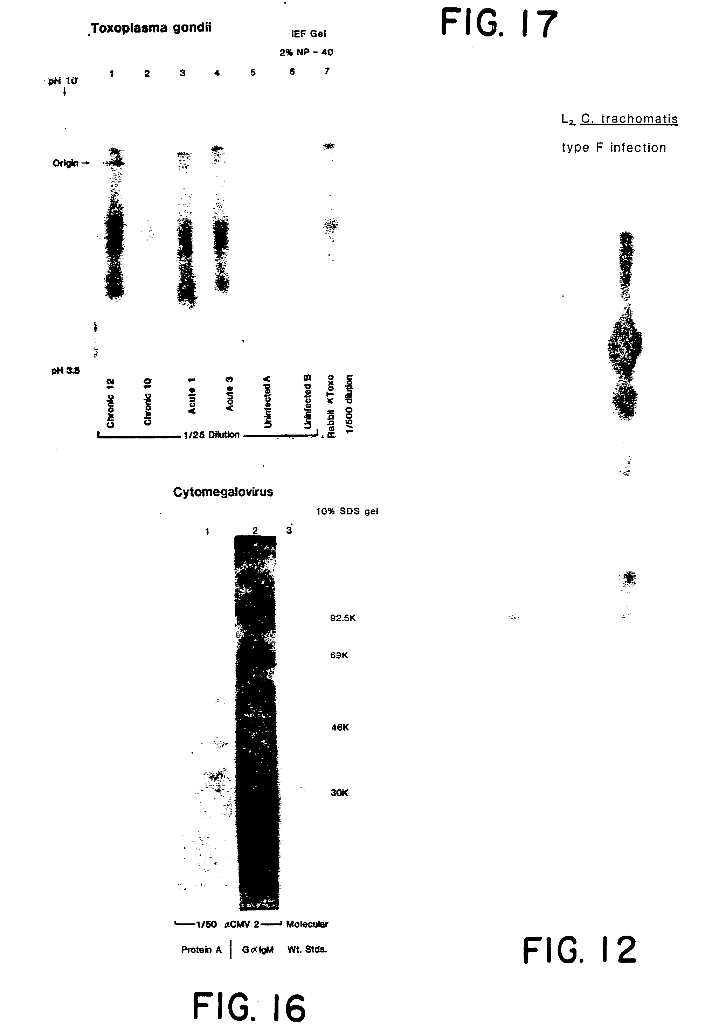

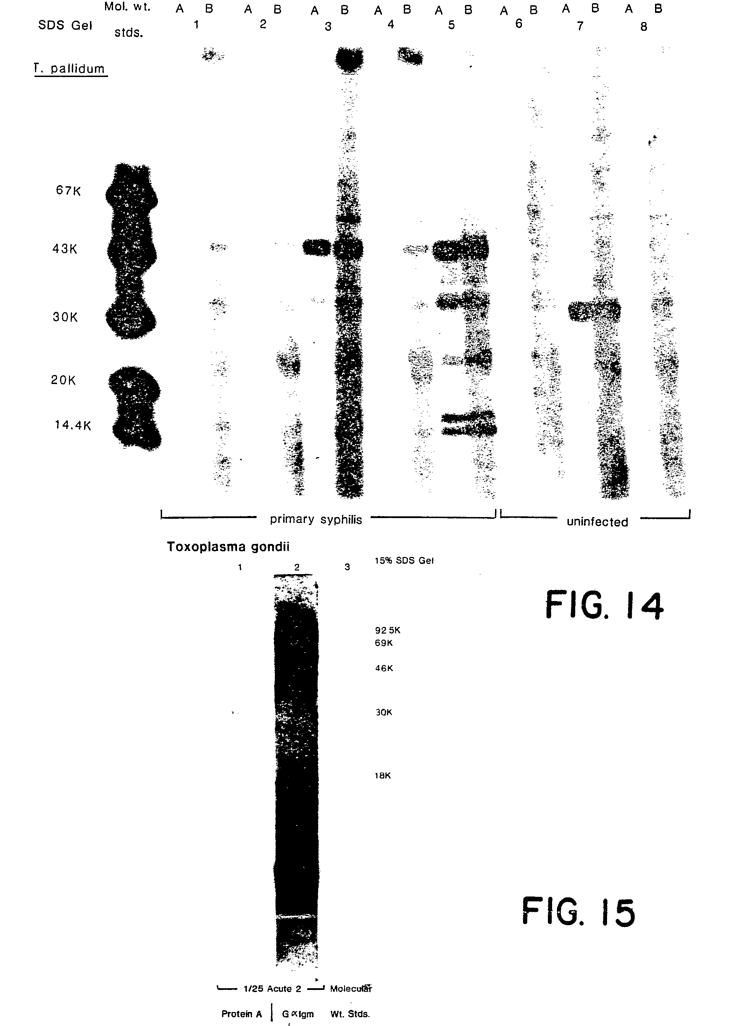

FIGURE 12 illustrates the reaction of serum from a patient with type F infection with proteins of type L2 *C. trachomatis.* FIGURE 13 shows, on an identical strip, the reaction of serum from a patient with type D infection with proteins of type L2. The similarities in the patterns are readily apparent. Unlike the microimmunofluorescence tests for *C. trachomatis*, where human infection with these immunotypes results in non-cross reactive surface antibody, these patterns show that the reaction is *C. trachomatis* species specific, not immunotype specific. Both strips were prepared and exposed in accordance with the procedures indicated in connection with Example I.

Example IX. Use of the Invention to Distinguish Between IgG and IgM Immunoglobulins

The diagnostic strips of the invention can be used to distinguish between classes of immunoglobulins. FIGURE 14 illustrates how IgM and IgG from syphilis patients may be readily distinguished, and how the presence of either of these antibodies may be distinguished from sera from normal patients. In FIGURE 14, *T. pallidum* is separated and stained on SDS polyacrylamide gel as is described in Example I. Rabbit antiserum to human IgM was labelled with $^{125}I$ and used as a probe for IgM. $^{125}I$ labelled Protein A of *S. aureus* was used as a probe for IgG.

The furthest right-hand column in the FIGURE 14 photograph is a standard system of molecular weight markers well known to those skilled in the art. The remaining sixteen columns represent diagnostic strips after exposure to serum from eight different patients. The right-hand strip (A) in each pair was probed with $^{125}I$-rabbit anti-human IgM and the left-hand strip (B) with $^{125}I$-Protein A, which is specific for IgG. The first five patients (i.e., the first ten strips) illustrate sera from patients with primary syphilis. The next three patients (i.e., the remaining six strips) illustrate sera from normal humans not infected with syphilis. Strips from these last three patients show that uninfected humans have little or no IgG or IgM antibodies to antigenic proteins derived from the organism that causes syphilis. In contrast, all patients with primary syphilis have IgG and IgM antibodies to the proteins from *T. pallidum*. The IgG or historic antibodies are clearly distinguishable from the current IgM antibodies in all the patients with primary syphilis.

FIGURE 15 shows that the diagnostic strips of the present invention can be used to distinguish between IgG and IgM antibodies in the sera of patients suffering from toxoplasmosis. *Toxoplasma gondii* were separated and run on SDS polyacrylamide gel, as is described in Example III; transfer of the gel protein pattern to the diagnostic strips is also described in that Example. Strips 1 and 2 were incubated with the serum from a patient with an acute case of toxoplasmosis. $^{125}I$ labelled Protein A of *S. aureus* was used as a probe for IgG in strip 1. $^{125}I$ labelled affinity-purified Goat antibodies to human IgM was used as a probe for IgM. Strip 3 represents well known molecular markers.

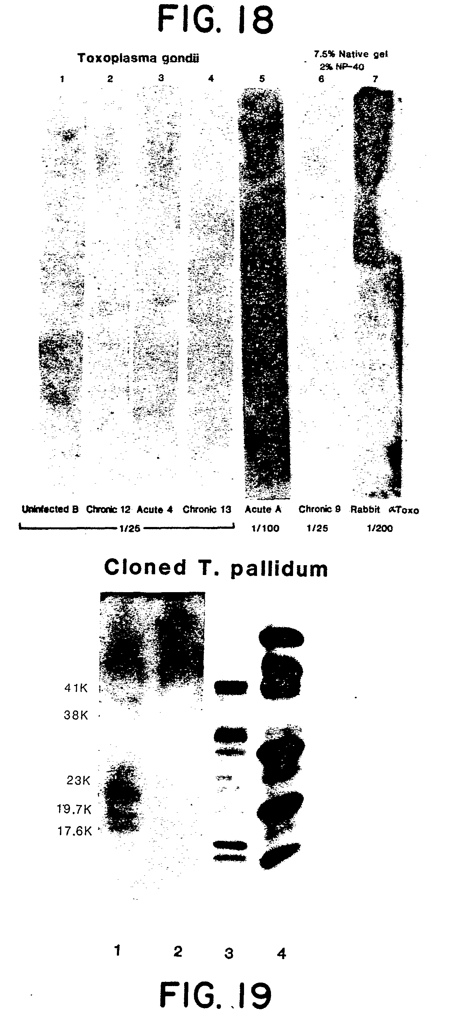

FIGURE 16 illustrates that the diagnostic strips can be used to distinguish between IgG and IgM antibodies in the sera of patients infected with cytomegalovirus. The samples and steps were prepared as outlined in Examples III and IV. Again, $^{125}I$ labelled Protein A was used as a probe for IgG and $^{125}I$ labelled affinity-purified Goat antibodies to human IgM were used as a probe for IgM. Strip 1 in FIGURE 16 shows the presence of IgG, strip 2 the presence of IgM. Strip 3 is a system of standard molecular markers.

Example X. Antigenic Proteins for the Diagnostic Strips Can be Separated on Nondenaturing Gels A. Use of nondenaturing isoelectricfocusing gel

FIGURE 17 illustrates the *Toxoplasma* antigen bands observed when the antigen is separated on nondenaturing isoelectricfocusing gel and sequentially incubated with patient sera and $^{125}I$ Protein A. A sonicate of *Toxoplasma gondii* is made 1% in noniodet P40. Nonsolubilized membranes are pelleted by centrifugation at 15,000 RPM for 2 minutes. The supernate is pipetted directly onto the pre-run gel.

The gel is made 5% in acrylamide, 0.0013% in bis acrylamide (T = 5.1%, C = 2.6%), 13% in sucrose, 2% noniodet P40, and 5% in ampholytes pH 3.5—10.0. The gel is polymerized with ammonium persulfate and TEMED for 1 hour. The gel is prerun for 1—2 hours of 30 ma constant current with a voltage maximum of 1000. The anode solution is 1M phosphoric acid, the cathode 1M sodium hydroxide. The samples are added to the gel and electrophoresed for 2.5 hours at 1000 volts. The separated antigens are transferred to cyanogen bromide treated paper as outlined in Example III, except that the gel is not washed with water and sodium acetate before transfer.

In FIGURE 17, strips 1—4 show the isoelectric bands from patients suffering from toxoplasmosis. Strips 5 and 6 are from uninfected humans and therefore show no bands. Strip 7 is a positive with a rabbit antiserum.

*B. Use of nondenaturing native gel*

FIGURE 18 shows the *Toxoplasma gondii* banding pattern obtained when antigens are separated on a non-denaturing native gel system and sequentially incubated with patient sera and $^{125}I$ Protein A. The procedure described in Example III for electrophoresis and transfer of *Toxoplasma* is utilized with the following modifications. The gel is made 7.5% in acrylamide, 0.2% in bis acrylamide, 2% in noniodet P40, and 75 mM trizma base plus 32 mM boric acid pH 8.9. The gel is polymerized with ammonium persulfate and TEMED for 20 minutes. The gel is overlayed with a stocking gel made 4% in acrylamide, 0.1% in bis acrylamide, 2% noniodet P40, and 37.5 mM trizma base plus 16 mM boric acid pH 8.9. This stacking gel is polymerized with ammonium persulfate and TEMED for 10 minutes. The electrode buffer for the system is 0.1% noniodet P40, 75 mM trizma base, 32 mM boric acid pH 8.9. The *Toxoplasma* sonicated organisms are made 1% in noniodet P40 and applied to the gel as in Example III.

Strip 1 is from an uninfected patient and therefore shows no bands characteristic of *Toxoplasma* antigens. Strips 2—6 are from patients suffering from various forms of toxoplasmosis; all show bands characteristic of the disease. Strip 7 is a positive control with a rabbit antiserum.

Example XI. The Antigenic Proteins of the Invention can be Produced by Genetically Engineered Microorganisms

The antigenic proteins used in the invention can be products of genes derived from antigenic organisms that have been separately cloned into suitable genetically engineered host microorganisms. Expression of cloned *T. pallidum* DNA in *E. coli* illustrates such antigenic protein production.

In this Example, *Treponema pallidum* were first harvested from the testicles of ten rabbits. The testicles were extensively minced in phosphate-buffered saline before the resulting extract was subjected to several cycles of differential centrifugation to remove cellular debris. The final supernatant, which contained motile and virulent *T. pallidum*, was further purified on a density gradient using a homogenous solution of Percoll, produced by Pharmacia Corporation, Piscataway, New Jersey 08854. Centrifugation at 20,000 RPM for 20 minutes produced a band of relatively pure, motile and virulent *T. pallidum*. The band was pulled from the Percoll gradient material, subjected to a dilution in phosphate-buffered saline, and then pelleted by ultracentrifugation at $100,000 \times G$ for 2 hours. The pellet of *T. pallidum* was resuspended in buffer containing tris-EDTA, pH 7.5, before treatment with the detergent Sarcosyl, (N-lauroylsarcosine) produced by Sigma Cehmicals, St. Louis, Missouri 63178, to liberate the treponemal DNA. The resulting DNA-detergent extract was centrifuged to equilibrium on a cesium chloride density gradient. The treponemal DNA band was then pulled from the gradient and dialysed against *Sau*3A I restriction buffer minus magnesium. The dialyzed DNA was partially digested with *Sau*3A I restriction endonuclease using techniques well known to those skilled in the art, and then ligated to purified *Bam*H I-cut arms of coliphage Charon 30. Rimm, D. L, et al, *Gene* 12:301—309 (1980). Ligation procedures were again those well known to those skilled in the art of recombinant DNA. The *T. pallidum* DNA-coliphage Charon 30 construct was packaged *in vitro,* Blattner, F. R., et al., *Science* 202:1279—1284 (1978) and then used to infect *E. coli* strain K 802. The resulting plaques were screened for *T. pallidum* antigens by an *in situ* radioimmunoassay. Screening was done by a modification of the "Western" blotting procedure of Towbin, H., et al., *PNAS* USA 76:4350—4354 (1979). Nitrocellulose discs were laid over the phage plaques, and the discs allowed to absorb protein for 10—30 minutes. Little protein was absorbed from unlysed *E. coli* of the lawn. The nitrocellulose filters were then coated with ovalbumin by soaking for 10 minutes in 5% ovalbumin in 50 mM tris-HCl (pH 7.5), 150 mM NaCl, 0.15% sodium azide (TSA—5%OA). The plaque blots were incubated overnight in either human secondary syphilitic sera or in normal human sera; both sera were diluted 1:300 in TSA—1%OA. Autoradiograms were prepared as described in Towbin, *supra,* after the blots were exposed to $^{125}I$-labelled *S. aureus* protein A.

One plaque, designated Tp3A, which gave a particularly strong reaction with a secondary syphilitic serum, was used for additional transformations. Phage from plaque Tp3A were diluted and replated on *E. coli* CSH 18. When rescreened with three different secondary syphilitic sera, all Tp3A plaques produced autoradiograms showing positive radioactive reactions. Autoradiograms from control plaques of cloning vector Charon 30 exhibited little or no radioactivity. This demonstrated that gene products from the Tp3A transformed hosts were antigenic for antibodies in sera of syphilitic individuals.

To further study the gene products from the Tp3A transformed hosts, a total protein lysate from the transformed hosts were submitted to SDS polyacrylamide gel electrophoresis as described in Example III. The differentiated polypeptides were then electrophoretically transferred to nitrocellulose strips as described in Example I. The strips were coated with ovalbumin and incubated with syphilitic sera. Again, autoradiograms were prepared as described in Example I after the blots were exposed to ¹²⁵I-labelled *S. aureus* protein A.

FIGURE 19 is a photograph of an autoradiogram of a diagnostic strip of cloned treponemal antigenic peptides from Tp3A transformed hosts. Strip 1 is the differentiated peptide patterns from the transformed hosts following exposure to syphilitic sera. Strip 2 is a Charon 30 control. Strip 3 shows the total *T. pallidum* protein profile after exposure to syphilitic sera. Strip 4 is standard system of molecular weight markers.

A comparison of strip 1 with strip 4 reveals that Tp3A genes code for at least five peptides of 41,000, 38,000, 23,000, 19,700, and 17,600 molecular weight which react specifically with syphilitic sera. The molecular weights of these cloned antigenic proteins correspond to the molecular weights of antigenic proteins of *T. pallidum* illustrated in strip 3. Control strip 2 shows that lysate proteins obtained from Charon 30 transformed hosts do not react with syphilitic sera. This demonstrates that the treponemal antigenic proteins are coded for by the cloned *T. pallidum* DNA.

It may be seen, therefore, that the invention provides in one aspect a method of diagnosing for the presence of a specific disease or a specific disease stage in a patient by which a much higher accuracy may be obtained in a very short time. The invention opens the possibility of providing physicians, in their offices or in small laboratories, with the ability to provide quick diagnosis on the basis of a sample of a patient's serum. Long waits and possible inaccuracies which are typical of many widely used clinical diagnosis techniques are eliminated. The technique of the invention is applicable to a wide variety of diseases,

merely requiring an initial series of comparison tests to ascertain and develop the empirical information necessary to select the optimum group of antigenic components and the optimum differentiation process.

Various modifications of the invention in addition to those shown and described herein will become apparent to those skilled in the art from the foregoing description. Such modifications are intended to fall within the scope of the appended claims.

## Claims

1. A method for producing an analytical antibody probe comprising disposing upon a solid support medium in a predetermined spatial relationship a set of differentiated antigenic components of a larger set of proteins and/or polysaccharides, said larger set being related to a disease or disease stage under analysis and said set of differentiated antigenic components being selected such that at least a subset thereof is known to be reactive with antibodies present in serum obtained from a patient having a specific disease or disease stage.

2. A method as claimed in claim 1, wherein the antigenic components are those of a pathogen, an allergen, tumour tissue, or a virus.

3. A method as claimed in claim 1, wherein the antigenic components are proteins, said proteins having been produced as the products of genes derived from an antigenic organism, which genes have been cloned separately into suitable genetically engineered host microorganisms.

4. A method as claimed in claim 3, wherein the antigenic organism is *Treponema Pallidum.*

5. A method as claimed in any one of claims 1 to 4, wherein the antigenic components are proteins which have been differentiated by means of gel electrophoresis.

6. A method as claimed in any one of claims 1 to 4, wherein the antigenic components are proteins which have been differentiated by means of non-denaturing gel system.

7. A method as claimed in claim 6, wherein the nondenaturing gel system is an isoelectric focusing gel system or a native gel system.

8. A method as claimed in any one of claims 5 to 7, wherein after differentiation the antigenic components thus differentiated are transferred from the gel to the solid support by means of filter affinity transfer.

9. An analytical antibody probe comprising a solid support medium upon which is disposed antigenic material related to a disease or disease stage, said antigenic material having been differentiated into distinct bands of antigenic components arranged in a relationship characteristic of said components.

10. A probe as claimed in claim 9 further defined by the feature or features of any one or more of claims 2 to 8.

11. The use of a probe produced by a method as claimed in any one of claims 1 to 8 or a probe as claimed in claim 9 or claim 10 for analysing a sample to determine whether it contains antibodies specific for antigenic material characteristic of a particular disease or disease stage by contacting said probe with said sample under conditions which permit reaction of any of said antibodies present in the sample with the differentiated antigenic components on the probe and detecting the existence and pattern of any antigen-antibody reactions on the probe, the probe antigenic components being related to the particular disease or disease stage under analysis.

## Revendications

1. Méthode de préparation d'un réactif analytique pour anticorps, caractérisé en ce que l'on dispose sur un milieu de support solide dans un arrangement spatial prédéterminé un ensemble de composants antigéniques différenciés d'un ensemble plus grand de protéines et/ou de polysaccharides, l'ensemble plus grand étant en rapport avec une maladie ou un état de maladie sous analyse et l'ensemble des composants antigéniques différenciés étant choisis de telle manière qu'au moins un sous-ensemble soit connu comme étant réactif avec les anticorps présents dans le sérum d'un malade ayant une maladie ou un état de maladie déterminés.

2. Méthode selon la revendication 1, caractérisé en ce que les composants antigéniques sont ceux d'un agent pathogène, un allergène, un tissu de tumeur ou un virus.

3. Méthode selon la revendication 1, caractérisée en ce que les composants antigéniques sont des protéines, ces protéines ayant été préparées en tant que produits de gènes dérivant d'un organisme antigénique, les gènes ayant été cloués séparément en des microorganismes convenables préparés par génie génétique pour servir d'hôtes.

4. Méthode selon la revendication 3, caractérisée en ce que l'organisme antigénique est *Treponema Pallidum.*

5. Méthode selon l'une quelconque des revendications 1 à 4, caractérisée en ce que les composants antigéniques sont des protéines qui ont été différenciées au moyen d'électrophorèse sur gel.

6. Méthode selon l'une quelconque des revendications 1 à 4, caractérisée en ce que les composants antigéniques sont des protéines qui ont été différenciées au moyen d'un système de gel non dénaturant.

7. Méthode selon la revendication 6, caractérisée en ce que le système de gel non dénaturant est un système de gel avec isoélectro-focalisation ou un système de gel natif.

8. Méthode selon l'une quelconque des revendications 5 à 7, caractérisée en ce que, après différenciation, les composants antigéniques ainsi différenciés sont transférés du gel au support solide au moyen d'un transfert par affinité de filtres.

9. Réactif analytique pour anticorps comprenant un milieu de support solide sur lequel est disposé un matériel antigénique en rapport avec une maladie ou un état de maladie, le matériel antigénique ayant été différencié en des groupes distincts de composants antigéniques agencés selon une disposition caractéristique des composants.

10. Réactif selon la revendication 9, défini en outre par les caractéristiques de l'une ou de plusieurs des revendications 2 à 8.

11. Utilisation d'un réactif préparé par une méthode selon l'une quelconque des revendications 1 à 8 ou un réactif selon la revendication 9 ou la revendication 10 pour l'analyse d'un échantillon afin de déterminer s'il contient des anticorps spécifiques pour un matériel antigénique caractéristique d'une maladie ou d'un état de maladie particuliers, par mise en contact du réactif avec l'échantillon dans des conditions permettant la réaction de l'un des anticorps présents dans l'échantillon avec les composants antigéniques différenciés sur le réactif et par détection de l'existence et des manifestations des réactions antigène-anticorps sur le réactif, les composants antigéniques du réactif étant en rapport avec la maladie ou l'état de maladie particuliers sous analyse.

**Patentansprüche**

1. Verfahren zur Herstellung eines analytischen Reagenz zur Bestimmung von Antikörpern, dadurch gekennzeichnet, daß man einen aufgetrennten Satz antigener Komponenten eines größeren Satzes von Proteinen und/oder Polysacchariden auf einem festen Trägermaterial in einem bestimmten räumlichen Verhältnis anordnet, wobei der größere Satz mit einer zu untersuchenden Krankheit oder einem zu untersuchenden Krankheitsstadium in Beziehung steht und der Satz aufgetrennter antigener Komponenten so ausgewählt ist, daß zumindest von einem Teil davon bekannt ist, daß er mit im Serum eines Patienten mit einer bestimmten Krankheit oder einem bestimmten Krankheitsstadium vorliegenden Antikörpern reagiert.

2. Verfahren nach Anspruch 1, wobei es sich um antigene Komponenten eines Pathogens, eines Allergens, eines Tumorgewebes oder eines Virus handelt.

3. Verfahren nach Anspruch 1, dadurch gekennzeichnet, daß die antigenen Komponenten Proteine sind, die als Produkte von aus einem antigenen Organismus abgeleiteten Genen hergestellt wurden, wobei die Gene in geeigneten genetisch manipulierten Wirts-Mikroorganismen getrennt cloniert wurden.

4. Verfahren nach Anspruch 3, wobei der antigene Organismus *Treponema Pallidum* ist.

5. Verfahren nach einem der Ansprüche 1 bis 4, wobei die antigenen Komponenten Proteine sind, die durch eine Gel-Elektrophorese aufgetrennt wurden.

6. Verfahren nach einem der Ansprüche 1 bis 4, wobei die antigenen Komponenten Proteine sind, welche mittels eines nicht-denaturierenden Gel-Systems aufgetrennt wurden.

7. Verfahren nach Anspruch 6, wobei das nicht denaturierende Gel-System ein isoelektrisches Fokusierungs-Gel-System oder ein natives Gel-System ist.

8. Verfahren nach einem der Ansprüche 5 bis 7, wobei die aufgetrennten antigenen Komponenten nach der Trennung vom Gel auf einen festen Träger mittels Filter-Affinitäts-Transfer übertragen werden.

9. Ein analytisches Reagenz zur Bestimmung von Antikörpern umfassend ein festes Trägermaterial, auf dem ein mit einer Krankheit oder einem Krankheitsstadium in Beziehung stehendes antigenes Material angeordnet ist, wobei das antigene Material in von einander unterscheidbaren Banden antigener Komponenten aufgetrennt wurde, die in einem für diese Komponenten typischen Verhältnis zueinander angeordnet sind.

10. Reagenz nach Anspruch 9, zusätzlich gekennzeichnet durch ein Merkmal oder Merkmale eines oder mehrerer der Ansprüche 2 bis 8.

11. Verwedenung eines nach einem Verfahren gemäß einem der Ansprüche 1 bis 8 hergestellten Reagenz oder eines Reagenz gemäß Anspruch 9 oder 10 bei der Untersuchung einer Probe, um zu bestimmen, ob diese für eine bestimmte Krankheit oder für ein bestimmtes Krankheitsstadium charakteristisches antigenes Material enthält, wobei das Reagenz mit der Probe unter Bedingungen in Berührung gebracht wird, die die Reaktion irgendeines der in der Probe vorliegenden Antikörpers mit den aufgetrennten antigenen Komponenten auf dem Reagenz erlauben und wobei das Auftreten sowie das Muster irgendeiner Antigen-Antikörper-Reaktion auf dem Reagenz nachgewiesen wird und wobei die antigenen Komponenten des Reagenz in Beziehung stehen mit der bestimmten Krankheit oder dem bestimmten Krankheitsstadium, das zu untersuchen ist.

Mol. Wt.

Stds.

SDS Gel

T. pallidum

1    2

# FIG. 1

FIG. 2

Mol.Wt.
Stds.

SDS Gel
T. pallidum

94K
67K
43K
30K
20K
14K

1  2  3  4  5  6  7  8  9

late Syphilis

secondary

# FIG. 3

FIG. 4

# FIG. 5

SDS Gel

Chlamydia

1    2    3    4    5  Mol. Wt.

Stds.

SDS Gel

Chlamydia

1     2     3     4     5

C.psittaci

# FIG. 6

**0 050 424**

**Toxoplasma gondii**                                          15% SDS Gel

| 1 | 2 | 3 | 4 | 5 | 6 | 7 | 8 |

92.5K
69K

46K

30K

18K

# FIG. 7

Chronic 19   Chronic 20   Acute 5   Acute 6   Negative A   Negative B   Chronic 12   Molecular

└────────────────────── 1/25 Dilution ──────────────────────┘ Wt. Stds.

Tissue                **Cytomegalovirus**                    Molecular
culture control ┌──── Infected tissue culture cells ── ┐ ┌── Viral prep.──┐   Wt. Std.
1        2        3        4          5          6          7              8

92.5K

69K

46K

# FIG. 8

30K

18K

1/100 αCMV #2        1/100 αCMV    1/100    1/100 αCMV    1/100

#1     CMV neg     #2      CMV neg

12% SDS Gel

7

FIG. 9

Streptococcus pneumoniae

Type 4

FIG. 10

Type 8

Normal          Immune

8

SDS Gel

L$_2$ C. trachomatis

bee venom

type D infection

## FIG. 13

## FIG. 11

0 050 424

FIG. 17

**Toxoplasma gondii**

IEF Gel

2% NP – 40

pH 10

1  2  3  4  5  6  7

L₂ _C. trachomatis_

type F infection

Origin →

pH 3.5

Chronic 12

Chronic 10

Acute 1

Acute 3

Uninfected A

Uninfected B

Rabbit αToxo 1/500 dilution

⌊_____ 1/25 Dilution _____⌋

**Cytomegalovirus**

10% SDS gel

1      2      3

92.5K

69K

46K

30K

⌞——1/50  αCMV 2——⌟  Molecular

Protein A │ G αIgM   Wt. Stds.

FIG. 16

FIG. 12

10

**0 050 424**

Mol. wt. A B A B A B A B A B A B A B A B

SDS Gel    stds.    1    2    3    4    5    6    7    8

Г. pallidum

67K

43K

30K

20K

14.4K

|————— primary syphilis —————|————— uninfected —————|

**Toxoplasma gondii**

1          2          3      15% SDS Gel

92 5K
69K

46K

30K

18K

|—— 1/25 Acute 2 ——|—— Molecular

Protein A | G αIgm    Wt. Stds.

# FIG. 14

# FIG. 15

11

# FIG. 18

Toxoplasma gondii  7.5% Native gel
2% NP-40

1    2    3    4    5    6    7

Uninfected B  Chronic 12  Acute 4  Chronic 13   Acute A   Chronic 9  Rabbit αToxo

├──────────── 1/25 ────────────┤   1/100    1/25     1/200

# Cloned T. pallidum

41K
38K

23K
19.7K
17.6K

1    2    3    4

# FIG. 19